# EUROPEAN PATENT APPLICATION

(11) **EP 4 772 203 A1**
(43) Date of publication of application: **08.07.2026**
(21) Application number: 24858512.7
(22) Date of filing: 26.08.2024
(51) Int. Cl.: A61L 27/42, A61L 27/56, A61L 27/58, A61L 27/12, C01B 25/32

(54) **ARTIFICIAL BONE REPAIR MATERIAL, POROUS MAGNESIUM-CONTAINING TRICALCIUM PHOSPHATE POWDER AND PREPARATION METHOD THEREFOR**

(30) Priority: 29.08.2023 CN 202311091421; 29.08.2023 CN 202311096242
(71) Applicant: Changzhou Bone-Renewal Medical Technologies LLC., Changzhou, Jiangsu 213000 (CN)
(72) Inventor: CUI, Wei, hangzhou, Jiangsu 213000 (CN); HONG, Dongli, hangzhou, Jiangsu 213000 (CN); ZHANG, Xing, hangzhou, Jiangsu 213000 (CN); CHEN, Jinqing, hangzhou, Jiangsu 213000 (CN)
(74) Representative: Bryers Intellectual Property Ltd
(86) International application number: PCT/CN2024/114504
(87) International publication number: WO 2025/044976

(57) **Abstract**

An artificial bone repair material and a preparation method therefor, and a preparation method for a porous magnesium-containing tricalcium phosphate powder for the artificial bone repair material. The artificial bone repair material is a composite material having a porous structure, and the raw materials of the artificial bone repair material comprise collagen and a porous magnesium-containing tricalcium phosphate powder, wherein the porous magnesium-containing tricalcium phosphate powder is formed by subjecting a precipitate obtained after the reaction of a calcium salt solution, a magnesium salt solution and a phosphate solution to spray drying, and the particle size of the magnesium-containing tricalcium phosphate powder is 1-20 µm. The mass percentage of magnesium-containing tricalcium phosphate in the artificial bone repair material is 50-95%. The artificial bone repair material has a relatively high ceramic component content, and the binding property and dispersity of the ceramic component in collagen are relatively good, such that the artificial bone repair material has relatively high biological activity and degradability, and the new bone tissue can grow inside until a bone defect part is completely replaced, while the artificial bone repair material is degraded in vivo.

## Description

### CROSS-REFERENCE TO RELATED APPLICATION

This application claims priority to Chinese patent applications filed on August 29, 2023, with application numbers CN2023110914215 and CN2023110962420, the entire contents of which are incorporated herein by reference.

### FIELD

The disclosure relates to a bone repair material, raw materials and a preparation method thereof, in particular to a porous magnesium-containing tricalcium phosphate and a collagen/magnesium-containing tricalcium phosphate artificial bone repair material prepared by taking collagen and porous magnesium-containing tricalcium phosphate as raw materials.

### BACKGROUND

With the deepening of population aging, the incidence of bone injuries is increasing year by year due to various factors including chronic diseases, traffic accidents, occupational injuries, and sports-related trauma. This creates an urgent need for novel artificial bone materials for bone defect repair. In recent years, a range of calcium phosphate-based artificial bone repair materials, represented by hydroxyapatite and tricalcium phosphate, have found widespread application. While the chemical composition resembles the inorganic component of human bone and offers good biocompatibility, calcium phosphate materials still present significant challenges, including non-degradability in vivo, a mismatch between the degradation rate and the osteogenesis rate, and low bioactivity. Consequently, existing materials struggle to satisfy the growing clinical demand.

Conventional bone repair materials predominantly utilize hydroxyapatite raw materials. While such materials demonstrate biocompatibility after the implantation, hydroxyapatite materials exhibit a slow degradation in vivo. Previous studies indicate that hydroxyapatite materials show no significant degradation even after several years of implanting to bone defects, thereby impeding the ingrowth of new bone tissue (Refer to reference 1: Dorozhkin S.V., Epple M. Biological and medical significance of calcium phosphates. Angew. Chem. Int. Ed .2002,41:3130-3146).

Dense hydroxyapatite only forms new bone tissue on the interface after implantation, and lacks the ability to induce bone formation. Compared with hydroxyapatite, tricalcium phosphate has better degradation performance, and can be chemically combined with bone after being implanted into the body, but it still lacks sufficient biological activity to further promote the formation of new bone. At the same time, tricalcium phosphate materials need to be prepared by high temperature calcination, which greatly increase the cost of bone repair materials (Refer to reference 2: Yadav M.K., Pandey V. and et al. A low-cost approach to develop silica-containing Tricalcium Phosphate (TCP) scaffold by valorizing animal bone waste and rice husk for tissue engineering applications. Ceram. Int. 2022,48:25335-25345).

Some researchers improve the biological activity of calcium phosphate materials by compounding collagen molecules. Collagen is an important component of human bone, which has excellent bone induction and can induce the proliferation and differentiation of bone cells (Refer to reference 3: Jiang Q.S., Wang L.R. and et al. Canine ACL reconstruction with an injectable hydroxyapatite/collagen paste for accelerated healing of tendon-bone interface. Bioact.Mater.2023,20:1-15).

However, it is not enough to provide biological activity only by collagen molecules. For the composite scheme of collagen and hydroxyapatite, the collagen powder and hydroxyapatite are mixed and hot-pressed at 90 - 130°C, and the product has obvious shortcomings in degradation performance (hydroxyapatite is difficult to degrade in vivo) and pore structure (hot-pressed and compact). The mainstream technologies in the industry also include collagen/hydroxyapatite composite freeze-drying molding method and collagen perfusion freeze-drying technology on the surface of porous tricalcium phosphate ceramics. Among them, the content of nano-hydroxyapatite powder used in collagen/hydroxyapatite composite freeze-drying molding method is 20-80%, which has poor dispersibility and cannot achieve higher ceramic phase content, and hydroxyapatite is difficult to degrade in vivo, which is not conducive to the formation and growth of new bones. However, the main body of the freeze-drying technology of pouring collagen on the surface of porous tricalcium phosphate ceramics is sintered calcium phosphate ceramics, and the matrix toughness is insufficient, so it is impossible to achieve the unique shaping technology of collagen.

### SUMMARY

An object of the disclosure provides an artificial bone repair material, which has a high content of ceramic components. The combination and dispersion of ceramic components in collagen are good, thus avoiding the problems of shedding and inflammation in the application process. The artificial bone repair material has high biological activity and biodegradability, and can degrade in vivo while the new bone tissue can grow in until the bone defect is completely replaced. Another object of the disclosure provides a preparation method of the porous magnesium-containing tricalcium phosphate powder for bone repair materials, with mild reaction conditions and reduced production costs. The low-density porous powder material produced has good adhesion with polymers such as collagen.

A first aspect of the present disclosure provides an artificial bone repair material, which is a composite material with a porous structure, and its raw materials include collagen and porous magnesium-containing tricalcium phosphate powder, which is formed by spray drying the precipitate after the reaction of calcium salt, magnesium salt and phosphate solution, and the particle size of the magnesium-containing tricalcium phosphate powder is 1 - 20 µm. The mass percentage of magnesium-containing tricalcium phosphate in the artificial bone repair material is 50 - 95%.

In an embodiment, the mass percentage of magnesium-containing tricalcium phosphate in the artificial bone repair material is 85 - 95%, that is, the content of ceramic components can reach more than 85%.

In an embodiment, the mass ratio of the collagen to the porous magnesium-containing tricalcium phosphate powder in the raw material is 0.05 - 1.

In an embodiment, the mass percentage of magnesium-containing tricalcium phosphate in the artificial bone repair material is 50 - 95%.

In an embodiment, the porosity of the artificial bone repair material is 70 - 95%.

In an embodiment, the artificial bone repair material is prepared by cross-linking and composite molding of collagen and porous magnesium-containing tricalcium phosphate powder.

In an embodiment, the mass percentage of magnesium in the porous magnesium-containing tricalcium phosphate powder is less than or equal to 3%.

In an embodiment, the porous magnesium-containing tricalcium phosphate powder is prepared by the following steps:
Preparing a mixed solution of calcium salt and magnesium salt as a first solution. Preparing a phosphate solution, and adjusting the pH to 9.5 - 10.5 with ammonia as a second solution.
Heating and mixing the first solution and the second solution, and reacting at 60 - 95°C with stirring.
Centrifugally separating the suspension obtained after the reaction to obtain precipitate, cleaning, and spray drying the cleaned precipitate to obtain porous magnesium-containing tricalcium phosphate powder.

In an embodiment, the lye is ammonia or sodium hydroxide.

A second aspect of the disclosure provides a preparation method of an artificial bone repair material, which comprises the following steps: dissolving collagen in an acid solution, adding porous magnesium-containing tricalcium phosphate powder, uniformly stirring to form a mixed slurry, and drying and molding to prepare the artificial bone repair material. Wherein, the porous magnesium-containing tricalcium phosphate powder is formed by spray drying the precipitate after the reaction of calcium salt, magnesium salt and phosphate solution, and the particle size is 1-20 µm.

In an embodiment, the preparation method comprises the following steps:
S101, dissolving collagen in an acid solution, adding porous magnesium-containing tricalcium phosphate powder, and uniformly stirring to prepare mixed slurry.
S102, adding a crosslinking agent into the mixed slurry, injecting into a molding die, and freeze-drying to obtain a molded composite material.
S103, removing the residual crosslinking agent in the composite material with eluent solution, washing with pure water for many times, and drying to obtain the artificial bone repair material.

In an embodiment, the mass ratio of collagen to porous magnesium-containing tricalcium phosphate powder is 0.05 - 1.

In an embodiment, the mass percentage of magnesium in the porous magnesium-containing tricalcium phosphate powder is less than or equal to 3%.

In an embodiment, the acid solution comprises a hydrochloric acid solution or an acetic acid solution with a concentration of 0.1 - 2 wt%.

In an embodiment, the concentration of the collagen in the acid solution is 0.5 - 5 wt%.

In an embodiment, the collagen comprises one or more combinations of type I collagen, type II collagen and recombinant collagen, and the molecular weight is 100000 - 350000 Da.

In an embodiment, step S102 is specifically implemented as follows: adding 1 - 100 ppm of cross-linking agent into the mixed slurry, injecting it into a molding die, vibrating the die to make the liquid level of the slurry level flat, then freezing the die in an environment of-4 - -80 °C, and then demoulding to obtain the molded composite material by a freeze-drying method. Wherein the crosslinking agent comprises one or a combination of two of glutaraldehyde and genipin, and the freeze-drying temperature is -20 - -80 °C.

In an embodiment, step S103 is specifically implemented as follows: soaking the composite material in an eluent solution for 0.5 - 24 hours, putting the eluted composite material into pure water, stirring and cleaning for 6 - 24 hours at the speed of 200 - 600 rpm, and then vacuum drying the composite material with water content greater than 90 wt% to obtain collagen/magnesium-containing tricalcium phosphate artificial bone repair material. Wherein the eluent comprises one or more combinations of polyvinyl alcohol and sodium borohydride, and the concentration is 0.05 - 3 wt.%. The vacuum degree of vacuumizing and drying treatment is 1 - 50 Pa, and the temperature of cold trap is -40 - -80 °C.

In an embodiment, the preparation method further comprises a preparation step of preparing the porous magnesium-containing tricalcium phosphate powder, which specifically comprises the following steps:
Preparing a mixed solution of calcium salt and magnesium salt as a first solution. Preparing a phosphate solution, and adjusting the pH to 9.5 - 10.5 with ammonia water as a second solution.
Heating and mixing the first solution and the second solution, and reacting at 60 - 95 °C with stirring.
Centrifugally separating the suspension obtained after the reaction to obtain precipitate, cleaning, and spray drying the cleaned precipitate to obtain porous magnesium-containing tricalcium phosphate powder.

In an embodiment, the molar ratio of calcium ions to magnesium ions in the first solution is 6 - 10. After the first solution and the second solution are mixed, the molar ratio of calcium ions to phosphate ions is 1.1 - 1.5. The calcium salt is selected from one or more combinations of anhydrous calcium chloride, calcium chloride dihydrate and calcium nitrate tetrahydrate, the magnesium salt is selected from magnesium chloride hexahydrate and/or magnesium nitrate hexahydrate, and the phosphate is selected from one or more combinations of disodium hydrogen phosphate, diammonium hydrogen phosphate and sodium hydrogen phosphate.

In an embodiment, the cleaned precipitate is spray-dried by spray drier, and the inlet air temperature of the spray drier is 240 - 300 °C, the outlet air temperature is 120 - 150 °C, and the feed rate is 30 - 200 ml/min.

Another aspect of the disclosure provides a preparation method of porous magnesium-containing tricalcium phosphate powder for artificial bone repair materials, comprising the following steps:
(i) Prepare a mixed solution of calcium salt and magnesium salt as the first solution. Prepare phosphate solution and adjust the pH to 9.5-10.5 with alkaline solution as the second solution.
(ii) Heat and mix the first solution and the second solution, and react under stirring conditions at 60-95 °C.
(iii) Centrifuge the suspension obtained after the reaction to collect and clean the sediment, and spray dry the cleaned sediment to obtain porous magnesium-containing tricalcium phosphate powder.

In an embodiment, the molar concentration ratio of calcium ions to magnesium ions in the first solution is 6-10.

In an embodiment, the molar concentration of calcium ions in the first solution is 0.01 - 0.9 mol/L, preferably 0.1 - 0.8 mol/L, more preferably 0.2 - 0.6 mol/L. For example, the molar concentration of calcium ions is 0.01 mol/L, 0.05 mol/L, 0.08 mol/L, 0.1 mol/L, 0.2 mol/L, 0.3 mol/L, 0.4 mol/L, 0.5 mol/L, 0.6 mol/L, 0.7 mol/L or 0.8 mol/L.

In an embodiment, the molar concentration of magnesium ions in the first solution is 0.001 - 0.15 mol/L, preferably 0.01 - 0.15 mol/L, more preferably 0.01 - 0.1 mol/L, further 0.05 - 0.08 mol/L. For example, the molar concentration of magnesium ions is 0.001 mol/L, 0.005 mol/L, 0.008 mol/L, 0.01 mol/L, 0.02 mol/L, 0.03 mol/L, 0.04 mol/L, 0.05 mol/L, 0.06 mol/L, 0.07 mol/L, 0.08 mol/L, 0.09 mol/L, 0.1 mol/L, 0.12 mol/L or 0.15 mol/L.

In an embodiment, the concentration of calcium salt is 1-100 g/L by weight, preferably 30-100 g/L, more preferably 50-100 g/L, further 60-90 g/L, particularly 80-90 g/L. For example, the concentration of calcium salt is 80 g/L, 82 g/L, 84 g/L, 86 g/L, 88 g/L or 90 g/L.

In an embodiment, the concentration of magnesium salt is 0.4-40 g/L, preferably 0.4-30 g/L, more preferably 5-30 g/L, further 15-30 g/L, particularly 20-30 g/L. For example, the concentration of magnesium salt is 20 g/L, 22 g/L, 24 g/L, 25 g/L, 27 g/L or 29 g/L.

In an embodiment, the molar ratio of calcium ions to phosphate ions after mixing the first solution and the second solution is 1.1-1.5.

In an embodiment, the molar concentration of phosphate in the second solution is 0.008-0.8 mol/L, preferably 0.01 - 0.8 mol/L, more preferably 0.05 to 0.8 mol/L, further 0.1 - 0.5 mol/L. For example, the molar concentration of phosphate is 0.008 mol/L, 0.009 mol/L, 0.01 mol/L, 0.02 mol/L, 0.03 mol/L, 0.04 mol/L, 0.05 mol/L, 0.06 mol/L, 0.07 mol/L, 0.08 mol/L, 0.09 mol/L, 0.1 mol/L, 0.2 mol/L, 0.3 mol/L, 0.4 mol/L, 0.5 mol/L, 0.6 mol/L, 0.7 mol/L or 0.8 mol/L.

In an embodiment, the concentration of phosphate in the second solution is 1-100 g/L by weight, preferably 30-100 g/L, more preferably 50-90 g/L, further 70-90 g/L, particularly 80-90 g/L. For example, the concentration of phosphate in the second solution is 81 g/L, 83 g/L, 84 g/L, 85 g/L, 87 g/L or 88 g/L.

In an embodiment, the first solution and the second solution are mixed in equal volumes.

In an embodiment, the solvent of the first solution is water, and the solvent of the second solution is water. In an embodiment, the solvent of the first solution comprises a combination of one or more selected from methanol, ethanol, and water, and the solvent of the second solution comprises a combination of one or more selected from methanol, ethanol, and water.

In an embodiment, the calcium salt comprises a combination of one or more selected from anhydrous calcium chloride, dihydrate calcium chloride, and tetrahydrate calcium nitrate, the magnesium salt comprises a combination of one or more selected from hexahydrate magnesium chloride and/or hexahydrate magnesium nitrate, and the phosphate salt comprises a combination of one or more selected from disodium hydrogen phosphate, diammonium hydrogen phosphate, and sodium hydrogen phosphate.

In an embodiment, the alkaline solution is ammonia water or sodium hydroxide.

In an embodiment, in step (i), the pH of the second solution is adjusted to 9.8-10.2 using ammonia water. More preferably, the pH of the second solution is 9.8-10.0.

In an embodiment, in step (ii), the first solution and the second solution are heated to 60-95 °C and mixed.

In an embodiment, in step (ii), the stirring rate is 100-500 rpm.

In an embodiment, in step (ii), the reaction is carried out under atmospheric pressure conditions.

In an embodiment, in step (iii), a spray dryer is used to dry the cleaned precipitate with spray. The inlet air temperature of the spray dryer is 240 - 300 °C, the outlet air temperature is 120 - 150 °C, and the feed rate is 30 - 200 mL/min.

In an embodiment, in step (iii), the precipitate is added to water for centrifugal cleaning for many times, and the washed precipitate is added to water with a mass of 5 - 20 times, stirred evenly to form slurry, and then dried by spray.

In an embodiment, the preparation method is implemented as follows:
Dissolve calcium salts and magnesium salts in water to obtain a first solution, where the concentration of calcium salts is 1-100 g/L and the concentration of magnesium salts is 0.4-40 g/L.
Dissolve phosphate in water and adjust the pH to 9.5-10.5 using ammonia water to obtain a second solution, with a concentration of phosphate ranging from 1-100 g/L.
Heat the first solution and the second solution with equal volume to 60-95 °C and mix. Under stirring conditions, react at atmospheric pressure to generate a suspension with a stirring rate of 100-500 rpm.
Centrifuge the suspension obtained after the reaction to collect the sediment, and centrifugally clean the sediment with water for 4 - 8 times, add the cleaned sediment into water with a mass of 5 - 20 times, stirred evenly to form slurry, and use a spray dryer to treat the slurry into micron porous tricalcium phosphate powder to obtain the bone repair raw material. The inlet air temperature of the spray dryer is 240 - 300 °C, the outlet air temperature is 120 - 150 °C, and the feed rate is 30 - 200 mL/min.

The artificial bone repair material of the disclosure uses degradable collagen and magnesium-containing tricalcium phosphate as raw materials, and realizes the design of total degradation in vivo. It is expected that the material will degrade in vivo and new bone tissue will grow in until the bone defect is completely replaced. Magnesium-containing tricalcium phosphate is a micron-sized low-density porous powder, which will not be separated by layers after being dispersed in collagen solution, which significantly increases the content of calcium phosphate ceramic components (50 - 95%), is evenly dispersed and has good adhesion, and avoids the problems of shedding and inflammation in the application process. At the same time, it has high activity, which can effectively promote osteogenic differentiation of bone tissue cells and promote new bone formation.

The preparation method of the porous magnesium-containing tricalcium phosphate powder disclosed in the disclosure is the wet chemical reaction of calcium/magnesium mixed solution and phosphate solution under the normal pressure at 60 - 95 °C to generate magnesium-containing tricalcium phosphate suspension, and then obtain low-density porous magnesium-containing tricalcium phosphate powder with a particle size of 1 - 20 µm through spray drying for shaping. The reaction conditions are mild and simple, avoiding the high-temperature of calcination process or solid phase reaction in traditional preparation techniques. The porous structure can provide better adhesion and dispersion in the preparation of bone repair materials, and promote the osteogenic differentiation of bone tissue cells and the generation of new bones. In the process of preparing bone repair materials with the powder, the special porous structure can form an interlocking structure between tricalcium phosphate and polymers (such as collagen), significantly improving the binding of the powder and solving the problems of shedding and inflammation that may occur during the application of nano tricalcium phosphate powder.

### DETAILED DESCRIPTION OF THE DRAWINGS

In order to provide a clearer explanation of the disclosed technical solution, a brief introduction will be given to the accompanying drawings required for the description of the embodiments. The drawings referenced in the following description correspond to selected embodiments of the present disclosure only. Additional figures may be derived therefrom by a person of ordinary skill in the art without the exercise of inventive faculty.
FIG. 1 depicts the X-ray diffraction (XRD) pattern of the porous magnesium-containing tricalcium phosphate powder obtained in Example 1 of the present disclosure.
FIG. 2 presents a scanning electron microscopy (SEM) micrograph of the porous magnesium-containing tricalcium phosphate powder produced in Example 1.
FIG. 3 illustrates a photographic image of the artificial bone-repairing material fabricated in Example 1.
FIG. 4 provides a SEM micrograph of the artificial bone-repairing material prepared in Example 1.
FIG. 5 is an interpenetrating microstructural representation of the porous magnesium-containing tricalcium phosphate and collagen phases within the artificial bone-repairing material of Example 1.
FIG. 6 displays a photographic image of the powder and debris suspended in deionized water after the artificial bone-repairing material from Comparative Example 1 was immersed in deionized water for one hour and subjected to fifty repetitive extrusion cycles.
FIG. 7 illustrates the mixed solution prepared in Comparative Example 2, wherein pronounced stratification is observed.
FIG. 8(a) and 8(b) are micrographs of MC3T3-E1 osteogenic precursor cells cultured for one day and three days, respectively, in the presence of the artificial bone-repairing material prepared in Example 1. FIG. 8(c) and 8(d) are micrographs of MC3T3-E1 osteogenic precursor cells cultured for one day and three days, respectively, in a blank control group.
FIG. 9 is the XRD pattern of the powder material prepared in Example 4 of the present disclosure.
FIG. 10 is the XRD pattern of the powder material prepared in Comparative Example 3.
FIG. 11 is the XRD pattern of the powder material prepared in Comparative Example 4.

### DETAILED DESCRIPTION OF THE EMBODIMENTS

The preferred embodiments of the present disclosure will be described in detail with reference to the accompanying drawings, so that the advantages and features of the present disclosure can be more easily understood by those skilled in the art. It should be noted here that the description of these embodiments is used to help understand the disclosure, but it does not constitute a limitation of the disclosure. In addition, the technical features involved in various embodiments of the present disclosure described below can be combined with each other as long as they do not conflict with each other.

The artificial bone repair material of the disclosure is a composite material with a porous structure including collagen and porous magnesium-containing tricalcium phosphate powder. The porous magnesium-containing tricalcium phosphate powder is formed by spray drying the precipitate after the reaction of calcium salt, magnesium salt and phosphate solution, and the particle size of the magnesium-containing tricalcium phosphate powder is 1 - 20 µm. The mass percentage of magnesium-containing tricalcium phosphate in the artificial bone repair material is 50 - 95%. Preferably, the mass percentage of magnesium-containing tricalcium phosphate in the artificial bone repair material is 70 - 95%. More preferably, the mass percentage of magnesium-containing tricalcium phosphate in the artificial bone repair material is 85 - 95%.

The mass ratio of the collagen to the porous magnesium-containing tricalcium phosphate powder in the raw materials is 0.05 - 1. The collagen comprises one or more combinations of type I collagen, type II collagen and recombinant collagen, and the molecular weight is 100000 - 350000 Da.

Further, the artificial bone repair material is prepared by cross-linking and molding collagen and porous magnesium-containing tricalcium phosphate powder. The crosslinking agent comprises one or a combination of glutaraldehyde and genipin.

Further, the mass percentage of magnesium in the porous magnesium-containing tricalcium phosphate powder is less than or equal to 3%, preferably 1 - 2%, and more preferably 1.3 - 1.9 wt%.

In the existing composite scheme of collagen and calcium phosphate material, the main component is hydroxyapatite, which is quite different from the idea of full degradation and regeneration in this application. However, if the degradable tricalcium phosphate material is used, most of it can only be prepared by high temperature calcination, and the micron-sized dense particles formed are difficult to disperse stably in collagen solution, so it is difficult to effectively compound with collagen. The active tricalcium phosphate powder material in this application is micron-sized low-density porous powder, which ensures the uniform dispersion of powder in collagen and significantly improves the content of calcium phosphate ceramic powder (50 - 95%).

Compared with hydroxyapatite, tricalcium phosphate has better degradation performance, so it has obvious advantages in the application of bone repair materials. Tricalcium phosphate raw materials usually need to be prepared by high-temperature calcination or solid-state reaction above 1000 °C, which consumes a lot of energy, and the product particles are micron-level with high density and irregular surface. At present, although some manufacturers can stably produce raw materials of tricalcium phosphate powder, most of them calcine the raw materials by solid-state reaction and crush the powder by mechanical grinding, which leads to higher cost, larger grains after sintering and irregular geometric shape, and poor fluidity in the process of preparing liquid-phase composite materials. The magnesium-containing tricalcium phosphate powder in this application is prepared based on wet chemical reaction, which directly generates magnesium-containing tricalcium phosphate suspension by wet chemical reaction at 60 - 95°C under normal pressure, and obtains low-density porous magnesium-containing tricalcium phosphate powder with a size of 1 - 20 µm through a special molding method of spray drying. This special micron-sized porous structure can make tricalcium phosphate powder form an interlocking structure with collagen in the process of preparing composite materials, and it is combined with collagen and has good dispersibility in collagen solution, which solves the problems of shedding and inflammation of nano tricalcium phosphate powder in the application process, and has important practical application value.

This application overcomes the problem of preparing tricalcium phosphate at low temperatures. By regulating the ion binding pathway in liquid-phase reactions, a low-temperature chemical synthesis process for tricalcium phosphate has been developed with reaction temperatures as low as 60-95 °C, resulting in a significant reduction of production costs by over 90%. Avoiding the harsh reaction conditions of high-temperature calcination (above 1000 °C) and hydrothermal synthesis (160-250 °C, 1.5 MPa). Secondly, the powder material prepared in this application is doped with active magnesium elements, which can effectively promote osteogenic differentiation of bone tissue cells and promote new bone formation. In response to the problems of weak binding force and poor dispersibility of tricalcium phosphate materials in the application process, the active tricalcium phosphate material synthesized by low-temperature wet chemical method in this application forms a micron sized low-density porous powder through spray drying process. This special porous structure can enable the tricalcium phosphate powder to form an interlocking structure with polymers such as collagen during the preparation of bone repair materials with good dispersibility and binding properties, solving the problems of shedding and inflammation that may occur in the application of nano tricalcium phosphate powder, and has important practical application value.

The artificial bone repair material of the disclosure is prepared by the following methods: dissolving collagen in an acid solution, adding porous magnesium-containing tricalcium phosphate powder, uniformly stirring to form mixed slurry, and drying and molding to prepare the artificial bone repair material. Wherein, the porous magnesium-containing tricalcium phosphate powder is formed by spray drying the precipitate after the reaction of calcium salt, magnesium salt and phosphate solution, and the particle size is 1 - 20 µm.

The preparation method specifically comprises the following steps:
S101, dissolving collagen in an acid solution, adding porous magnesium-containing tricalcium phosphate powder, and uniformly stirring to prepare mixed slurry.
S102, adding a crosslinking agent into the mixed slurry, injecting into a molding die, and freeze-drying to obtain a molded composite material.
S103, removing the residual crosslinking agent in the composite material with eluent solution, washing with pure water for many times, and drying to obtain the artificial bone repair material.

In step S101, the mass ratio of collagen to porous magnesium-containing tricalcium phosphate powder is 0.05 - 1, preferably 0.1 - 0.4, and more preferably 0.1 - 0.2. For example, the mass ratio of collagen to porous magnesium-containing tricalcium phosphate powder can be 0.05, 0.08, 0.1, 0.2, 0.3, 0.4 or 0.5.

The acid solution comprises hydrochloric acid solution and/or acetic acid solution with a concentration of 0.1 - 2 wt%. The concentration of collagen in the acid solution is 0.5 - 5 wt%, preferably 1 - 5 wt%, more preferably 1 - 3 wt%, further 1.2 - 2 wt%. For example, the concentration of collagen in the acid solution is 0.5 wt%, 1.0 wt%, 1.2 wt%, 1.5 wt%, 2 wt%, 3 wt% or 5 wt%. Collagen includes one or more combinations of type I collagen, type II collagen and recombinant collagen, and its molecular weight is 100000 - 350000 Da.

Step S101 is specifically implemented as follows: using an analytical balance to weigh collagen and micron-sized low-density porous magnesium-containing tricalcium phosphate powder. Weigh the acid solution with a balance, add all the collagen into the solution, and stir with a polytetrafluoroethylene paddle at 300 - 1200 rpm until the collagen is completely dissolved. Then the micron-sized low-density porous magnesium-containing tricalcium phosphate powder is completely added into the collagen solution, and the mixture is continuously stirred for 5 - 30 minutes until it is evenly mixed. Wherein, the used porous magnesium-containing tricalcium phosphate powder is prepared by the following steps:
(1) Dissolving calcium salt and magnesium salt in water to obtain a first solution, wherein the concentration of calcium salt is 1 - 100 g/L and the concentration of magnesium salt is 0.4 - 40 g/L.. Further, the molar ratio of calcium ions to magnesium ions is 6 - 10. The concentration of calcium salt is more preferably 30 - 100 g/L, further 50 - 100 g/L, further 60 - 90 g/L, especially 80 - 90 g/L. For example, 80 g/L, 82 g/L, 84 g/L, 86 g/L, 88 g/L or 90g/L. Optionally, the concentration of magnesium salt is more preferably 0.4 - 30 g/L, further 5 - 30 g/L, further 15 - 30 g/L, especially 20 - 30 g/L. For example, 20 g/L, 22 g/L, 24 g/L, 25 g/L, 27 g/L or 29 g/L.
(2) Dissolving phosphate in water, and adjusting the pH to 9.5 - 10.5, further 9.8 - 10.0 by using ammonia water to obtain a second solution, wherein the concentration of phosphate is 1 - 100 g/L. The concentration of phosphate in the second solution is more preferably 30 - 100 g/L, further 50 - 90 g/L, further 70 - 90 g/L, especially 80 - 90 g/L. For example, 81 g/L, 83 g/L, 84 g/L, 85 g/L, 87 g/L or 88 g/ L. More preferably, ammonia water is used to adjust it to 9.8 - 10.2. The inventors found that this pH value has an important influence on the preparation of porous magnesium-containing tricalcium phosphate powder. According to the X-ray diffraction results, too high or too low pH value will produce more impurity phases, resulting in more calcium hydrogen phosphate impurity phases or hydroxyapatite in the prepared porous magnesium-containing tricalcium phosphate powder.
(3) The first solution and the second solution with the same volume are heated to 60 - 95°C and then mixed, and wet chemical reaction is carried out at 60 - 95°C under stirring conditions at normal pressure, wherein the stirring speed is 100 - 500 rpm, and the molar ratio of calcium ions to phosphate ions is 1.1 - 1.5.
(4) Centrifugally separating the suspension after wet chemical reaction to obtain precipitate, centrifugally cleaning it with water for 4 - 8 times, adding the washed precipitate into water with 5-20 times the mass, uniformly stirring to form slurry, and treating the rubber compound into micron - sized porous tricalcium phosphate powder by using spray drier to obtain the bone repair raw material, wherein the air inlet temperature of spray drier is 240 - 300°C, the air outlet temperature is 120 - 150°C, and the feed rate is 30 - 200 mL/min.

The calcium salt is selected from one or more combinations of anhydrous calcium chloride, calcium chloride dihydrate and calcium nitrate tetrahydrate, the magnesium salt is selected from magnesium chloride hexahydrate and/or magnesium nitrate hexahydrate, and the phosphate is selected from one or more combinations of disodium hydrogen phosphate, diammonium hydrogen phosphate and sodium hydrogen phosphate.

The micron-sized porous tricalcium phosphate powder has a particle size of 1 - 20 µm and a porous microstructure, in which the content of magnesium is 1 - 2 wt%, preferably 1.3 - 1.9 wt%, showing excellent biological activity and effectively promoting new bone formation.

In step S102, the crosslinking agent comprises one or a combination of glutaraldehyde and genipin, and the concentration of the crosslinking agent in the mixed slurry is 1 - 100 ppm.

Step S102 is specifically implemented as follows: adding 0.1 - 10 ppm cross-linking agent into the mixed slurry, injecting 1 - 50 ml into a mold with a bottom area of 1 - 25 cm², and vibrating the mold to make the liquid level of the slurry smooth. Then the molding die is put into a refrigerator at-4 - -80°C for freezing, and then the mold is demolded and the molded composite material is preliminarily obtained by a freeze-drying method. Among them, the freeze-drying temperature is -20 - -80°C, and the mold material includes but is not limited to silica gel, PTFE, medical stainless steel, etc.

Step S103 is specifically implemented as follows: soak the composite material in eluent solution for 0.5 - 24 hours, put the eluted composite material in pure water at 200 - -600 rpm, stir and clean it for 6-24 hours, and then vacuum dry the composite material with water content of 90-97 wt% to obtain collagen/magnesium-containing tricalcium phosphate artificial bone repair material. Among them, the elution reagent includes but is not limited to polyvinyl alcohol, sodium borohydride, etc., and the concentration is 0.05 - 3 wt%. The vacuum degree in the process of vacuumizing and drying is 1 - 50 Pa, and the temperature of the cold trap is -40 - -80°C.

The prepared collagen/magnesium-containing tricalcium phosphate artificial bone repair material has a porosity of 70-95%, a maximum pore size of more than 300 µm, and a magnesium-containing tricalcium phosphate content of 50-95 wt%, preferably 85-95 wt%.

### Example 1

### (1) Fabrication of Porous Magnesium-Containing Tricalcium Phosphate Powder

Weighing and Dissolution: Solution A was prepared by dissolving 900 g anhydrous calcium chloride and 270 g magnesium chloride hexahydrate in 10 L deionized water. Concurrently, solution B was formed through dissolution of 830 g diammonium hydrogen phosphate in 10 L deionized water, with subsequent pH adjustment to 10.0 using ammonium hydroxide.

Thermal Mixing: Both solutions A and B were preheated to 70°C and combined under continuous agitation at 100 rpm, resulting in a homogeneous suspension.

Hydrothermal Reaction: The mixed suspension underwent isothermal stirring at 70°C and 100 rpm for 12 hours.

Centrifugal Purification: The reaction suspension was centrifuged at 2000 rpm for 3 minutes to isolate precipitate. Five successive centrifugal washing cycles with deionized water were performed.

Spray Drying: Purified precipitate was resuspended in 10 L deionized water to form slurry. Spray drying was conducted at 50 mL/min feed rate with 250°C inlet and 120°C outlet temperatures, yielding micron-scale low-density porous tricalcium phosphate powder exhibiting 1-20 µm particle size distribution.

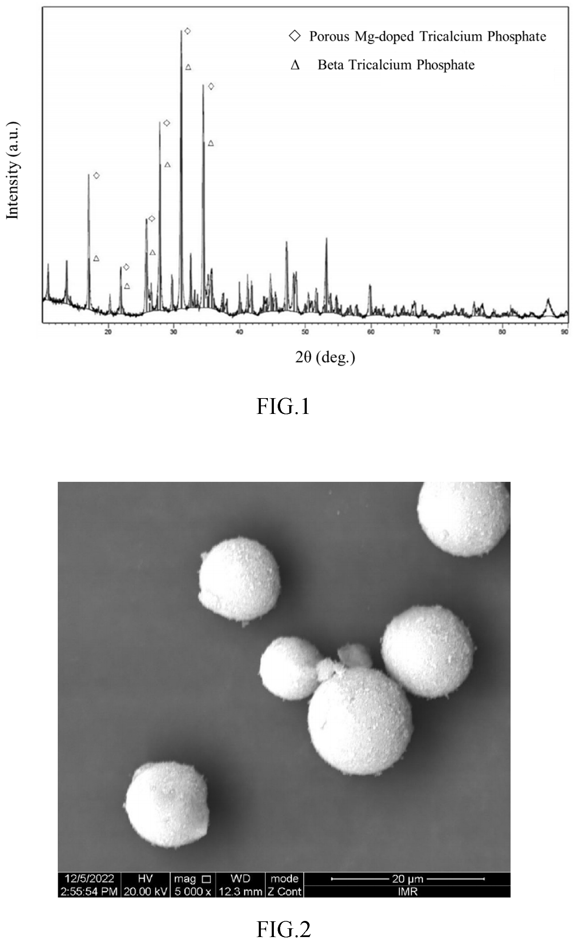

Characterization: The X-ray diffraction results of micrometer low-density porous magnesium-containing tricalcium phosphate powder are shown in Figure 1, where the mass percentage of magnesium element is 1.9%. Scanning electron microscopy (Figure 2) revealed characteristic porous microstructure. This unique porosity facilitates interlocking structural integration with polymeric matrices during composite fabrication.

### (2) Synthesis of Artificial Bone Repair Material

Weighing and Dissolution: A collagen solution was prepared by dissolving 0.4 g Type I collagen in 20 g 0.1% hydrochloric acid solution under 400 rpm tetrafluoroethylene paddle agitation for 1 hour. Separately, 2.4 g porous magnesium-containing tricalcium phosphate powder synthesized from section (1) was weighed.

Mixing Configuration: The total tricalcium phosphate powder was incorporated into the collagen solution. Homogeneous dispersion was achieved through 10 minutes stirring at 400 rpm using a tetrafluoroethylene paddle.

Cross-Linking and Molding: Twenty microliters of 25% glutaraldehyde solution was introduced into the mixture. After 1 minute agitation at 400 rpm, the solution was transferred to a PTFE mold and leveled by gentle oscillation. Cryofixation at -20°C for 6 hours preceded demolding and freeze drying at -70°C for 48 hours, yielding a preformed composite.

Purification: Residual crosslinkers were removed via 0.5 hour immersion in 1 wt% sodium borohydride solution. Sequential purification involved 24 hours washing with 10 times volume of purified water under 400 rpm agitation.

Freeze drying: Purified composites were frozen at -20°C for 6 hours and lyophilized for 36 hours to obtain the final collagen/magnesium-containing tricalcium phosphate artificial bone repair material.

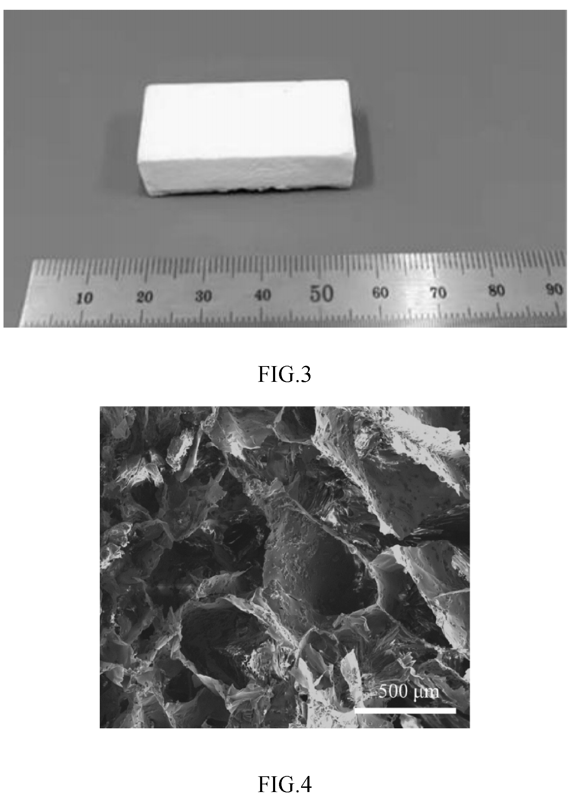

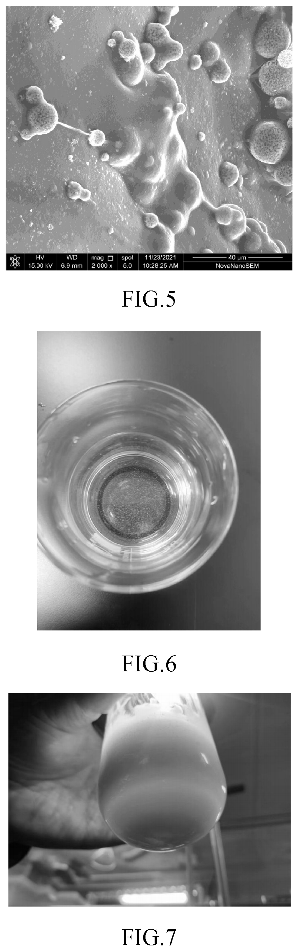

Structural Analysis: Figure 3 illustrates the microstructure of collagen-tricalcium phosphate bone repair materials with preserved porous microstructure of porous magnesium-containing tricalcium phosphate. Figure 4 demonstrates lamellar fiber microstructure formation through physicochemical synergistic crosslinking. Referring to Figure 5, a micro low-density porous magnesium-containing tricalcium phosphate powder forms an interpenetrating microstructure with collagen. The porous magnesium-containing tricalcium phosphate powder has good adhesion with collagen, avoiding the problems of detachment and inflammation that may occur during the application of tricalcium phosphate powder. The mass percentage of magnesium-containing tricalcium phosphate in artificial bone repair materials is 85%.

### Example 2

Weighing and Dissolution: A quantity of 0.25 g of Type I collagen was precisely weighed using an analytical balance. Concurrently, 20 g of 0.5% acetic acid solution was measured employing the same analytical balance. The total collagen mass was introduced into the acid solution, followed by homogenization through stirring at 1000 rpm for 0.3 hours using a tetrafluoroethylene paddle, resulting in a collagen solution. Separately, 1 g of porous magnesium-containing tricalcium phosphate powder synthesized according to Example 1 was weighed.

Mixing Configuration: The entirety of porous magnesium-containing tricalcium phosphate powder was incorporated into the collagen solution. Uniform dispersion was achieved by stirring at 1000 rpm for 10 minutes with a tetrafluoroethylene paddle, yielding a homogeneous mixed solution.

Cross-Linking and Molding: Ten microliters of 25% glutaraldehyde solution was introduced into the mixed solution. After agitation at 1000 rpm for 1 minute using a tetrafluoroethylene paddle, the solution was transferred into a silicone mold. Gentle manual oscillation was applied until liquid surface planarization was attained. Subsequent cryofixation was performed at -20°C for 6 hours in a low-temperature cabinet, followed by demolding and freeze drying at -70°C for 48 hours to obtain a preformed composite.

Purification: Residual crosslinking agents were eliminated through immersion of the composite material in 0.15 wt% sodium borohydride solution for 1 hour. A sequential washing procedure was then conducted utilizing 10 volumes of purified water under constant agitation at 400 rpm for 24 hours.

Freeze drying: Purified samples underwent freezing at -20°C for 6 hours with subsequent freeze drying for 36 hours, yielding the final collagen/magnesium-containing tricalcium phosphate artificial bone repair material. The mass percentage of magnesium-containing tricalcium phosphate in artificial bone repair materials is 80%.

### Example 3

Weighing and Dissolution: A measured quantity of 4 g of Type I collagen was weighed utilizing an analytical balance. Concurrently, 80 g of 0.1% dilute hydrochloric acid solution was quantified employing the same analytical balance. The entirety of the collagen was introduced into the acid solution. Homogenization was performed through stirring at 800 rpm for 0.5 hours using a tetrafluoroethylene paddle. Separately, 8 g of porous magnesium-containing tricalcium phosphate powder synthesized according to Example 1 was weighed.

Mixing Configuration: The total mass of porous magnesium-containing tricalcium phosphate powder was incorporated into the collagen solution. Uniform dispersion was achieved by stirring at 800 rpm for 10 minutes with a tetrafluoroethylene paddle, yielding a homogeneous mixed solution.

Cross-Linking and Molding: Forty microliters of 25% glutaraldehyde solution was introduced into the mixed solution. After 1 minute of stirring at 800 rpm with a tetrafluoroethylene paddle, the solution was transferred into a 316L medical-grade stainless steel mold. Gentle agitation was applied to attain liquid surface planarity. Subsequent cryofixation was conducted at -20°C for 6 hours in a low-temperature cabinet, followed by demolding and freeze drying at -70°C for 48 hours to obtain a preformed composite.

Purification: The composite material underwent immersion in 0.5 wt.% sodium borohydride solution for 1 hour to eliminate residual chemical crosslinking agents. This was followed by a 24-hour washing cycle with 10 volumes of purified water under constant agitation at 400 rpm.

Freeze drying and Final Product: Purified samples were subjected to freezing at -20°C for 6 hours and subsequent freeze drying for 36 hours, resulting in the final collagen/magnesium-containing tricalcium phosphate artificial bone repair material. The mass percentage of magnesium-containing tricalcium phosphate in artificial bone repair materials is 67%.

### Comparative Example 1

Weighing and Dissolution: A quantity of 0.4 g of Type I collagen was weighed using an analytical balance. Concurrently, 20 g of a 0.1% dilute hydrochloric acid solution was weighed employing the same analytical balance. The entirety of the weighed collagen was introduced into the hydrochloric acid solution. This mixture underwent stirring for 1 hour at 400 rpm utilizing a tetrafluoroethylene paddle, resulting in formation of a collagen solution. Separately, 2.4 g of magnesium-containing tricalcium phosphate nanopowder was weighed via analytical balance. said nanopowder was synthesized according to the methodology described in Example 1 of Chinese Patent ZL201610290891.8.

Mixing Configuration: The total quantity of weighed magnesium-containing tricalcium phosphate nanopowder was incorporated into the collagen solution. Homogenization was achieved through stirring for 10 minutes at 400 rpm using a tetrafluoroethylene paddle, yielding a uniformly dispersed mixed solution.

Cross-Linking and Molding: To the mixed solution, 20 microliters of 25% glutaraldehyde solution was added. After 1 minute of stirring at 400 rpm with a tetrafluoroethylene paddle, the solution was transferred into a silicone mold. The mold was gently agitated to achieve a leveled liquid surface. Subsequent freezing occurred at -20°C in a low-temperature cabinet for 6 hours, followed by demolding and freeze drying at -70°C for 48 hours to obtain a preliminarily formed composite.

Purification: The composite material was immersed in a 1 wt% sodium borohydride solution for 0.5 hour to eliminate residual crosslinking agents. This was followed by a 24-hour washing procedure with 10 volumes of purified water under constant stirring at 400 rpm.

Freeze drying: Purified samples were frozen at -20°C for 6 hours and subsequently lyophilized for 36 hours to yield the final composite material. As illustrated in Figure 6, when subjected to 50 cycles of repeated extrusion in purified water, the composite exhibited structural collapse and surface powder shedding.

### Comparative Example 2

Weighing and Dissolution: A quantity of 1.6 g of Type I collagen was weighed using an analytical balance. Concurrently, 80 g of a 0.1% dilute hydrochloric acid solution was weighed using the same analytical balance. The entirety of the weighed collagen was added to the hydrochloric acid solution. This mixture was stirred for 1 hour at 400 rpm using a tetrafluoroethylene paddle, resulting in the preparation of a collagen solution. Separately, 9.6 g of tricalcium phosphate was weighed using an analytical balance. this tricalcium phosphate was synthesized via a high-temperature calcination process at 1100°C.

Mixing and Configuration: The entirety of the weighed tricalcium phosphate powder was introduced into the collagen solution. This combined mixture was stirred for 10 minutes at 400 rpm using a tetrafluoroethylene paddle until homogeneous dispersion was achieved, yielding a mixed solution. As depicted in Figure 7, after allowing the mixed solution to stand undisturbed for 0.5 hours, a distinct delamination phenomenon was observed. Consequently, further processing for composite material fabrication could not be performed.

### Cell experiment of leaching solution

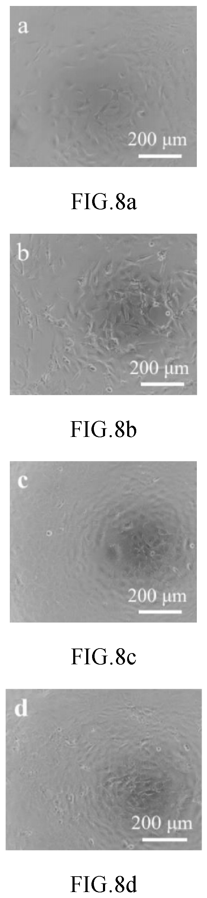

MC3T3-E1 osteoprogenitor cells were cultured separately in: (i) the collagen/porous magnesium-containing tricalcium phosphate composite extract prepared according to Example 1, and (ii) a blank control medium. Cellular morphology was microscopically examined after 1 day and 3 days incubation periods. Observation results are presented in Figures 8(a-d). The leachate cell experiment demonstrates a comparable cellular growth state between the Example 1 artificial restoration material and the control group, indicating absence of cytotoxicity and presence of biocompatibility.

The collagen/porous magnesium-containing tricalcium phosphate composite employs degradable collagen and tricalcium phosphate as primary constituents, achieving a fully degradable in vivo design. This material is anticipated to undergo gradual degradation while facilitating new bone tissue regeneration until complete defect replacement. Active magnesium elements contained within the magnesium-containing tricalcium phosphate effectively promote osteogenic differentiation of bone tissue cells and accelerate new bone formation. The porous powder structure of magnesium-containing tricalcium phosphate maintains homogeneity when dispersed in collagen solution. This characteristic enables significant increase in calcium phosphate ceramic powder content (65-95 wt%). The particulate material exhibits a 5-20 µm particle size range and retains its agglomerated state throughout composite processing.

### Example 4

Weighing and Dissolution: 1800 g of anhydrous calcium chloride and 400 g of magnesium chloride hexahydrate were weighed on an analytical balance and dissolved in 20 L of water to afford solution A. 1700 g of diammonium hydrogen phosphate were weighed on the same analytical balance, dissolved in 20 L of water, and the pH of the resulting solution was adjusted to 9.8 with aqueous ammonia to afford solution B.

Heating and Mixing: solution A and solution B were heated to 90 °C and mixed, and the mixture was continuously agitated at 100 rpm to generate a suspension.

Wet Chemical Reaction: the suspension obtained from the mixing step was maintained at 90 °C and stirred at 100 rpm for 4 hours.

Centrifugal Cleaning: the suspension obtained after the wet chemical reaction was centrifuged at 2000 rpm for 3 minutes to collect a precipitate, and the precipitate was centrifugal washed with water five times.

Spray Drying: the washed precipitate was dispersed in 20 L of water and homogenized by stirring to form a slurry. A spray-dryer was operated with an inlet temperature of 270 °C and an outlet temperature of 140 °C, and the slurry was spray-dried at a feed rate of 100 mL/min to yield micron-sized, low-density porous tricalcium phosphate powder exhibiting a particle-size distribution within the range of 1 µm to 20 µm.

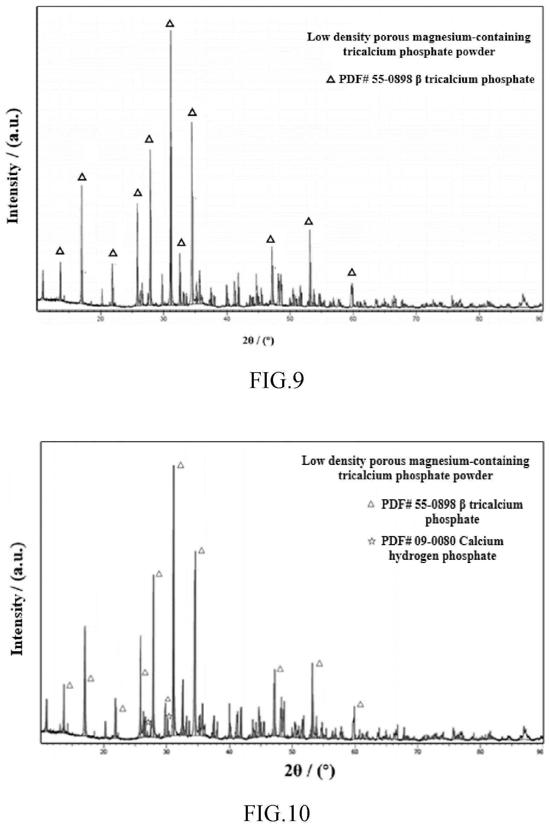

Characterization: the micron-sized, low-density porous tricalcium phosphate powder exhibited the X-ray diffraction pattern shown in FIG. 9 and the magnesium content is 1.3 %.

### Comparative Example 3

Weighing and Dissolution: 1800 g of anhydrous calcium chloride and 300 g of magnesium chloride hexahydrate were weighed by an analytical balance and dissolved in 20 L of water to provide solution A. 1700 g of diammonium hydrogen phosphate were weighed on the same analytical balance, dissolved in 20 L of water, and the pH of the resulting solution was adjusted to 9.3 with aqueous ammonia to provide solution B.

Heating and Mixing: solution A and solution B were heated to 90 °C and combined, and the mixture was continuously agitated at 100 rpm to generate a suspension.

Wet Chemical Reaction: the suspension obtained from the mixing step was maintained at 90 °C and stirred at 100 rpm for 4 hours.

Centrifugal Cleaning: the suspension obtained after the wet chemical reaction was centrifuged at 2000 rpm for 3 minutes to collect a precipitate, and the precipitate was centrifugal washed with water five times.

Spray Drying: the washed precipitate was dispersed in 20 L of water and homogenized by stirring to form a slurry; a spray-dryer was operated with an inlet temperature of 270 °C and an outlet temperature of 140 °C, and the slurry was spray-dried at a feed rate of 100 mL/min to yield micron-sized, low-density porous tricalcium phosphate powder.

Characterization: the tricalcium phosphate powder exhibited the X-ray diffraction pattern shown in FIG. 10, wherein the porous tricalcium phosphate powder contained impurity phases, primarily dicalcium phosphate.

### Comparative Example 4

Comparative Example 4 differed from Example 4 solely in that, after diammonium hydrogen phosphate was dissolved in water, the pH of the resulting solution was adjusted to 10.8 with aqueous ammonia, and all other procedural steps remained identical.

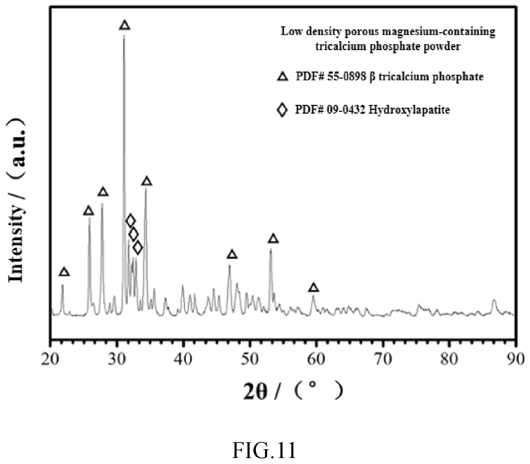

X-ray diffraction analysis of the obtained powder material, as depicted in FIG. 11, revealed the presence of a significant amount of hydroxyapatite in the powder produced by Comparative Example 4.

As shown in this specification and claims, the terms "including" and "containing" only indicate the steps and elements that have been clearly identified, and these steps and elements do not constitute an exclusive listing. Methods or devices may also contain other steps or elements. The term 'and/or' used in this disclosure includes any combination of one or more related listed items.

The endpoints and any values disclosed in this disclosure are not limited to the exact range or value, and these ranges or values should be understood as including values close to these ranges or values. For numerical ranges, the endpoint values of each range, the endpoint values of each range and individual point values, and individual point values can be combined with each other to obtain one or more new numerical ranges, which should be considered as specifically disclosed in this article.

Unless otherwise defined, all technical and scientific terms used herein have the same meaning as commonly understood by people having ordinary skill in the art. If there is any contradiction or inconsistency between the definition used in this disclosure and the definition contained in other public literature, the definition used here shall prevail.

The above embodiments are only intended to illustrate the technical concept and characteristics of this disclosure, and are a preferred embodiment. The purpose is for those in the art to understand the content of this disclosure and implement it accordingly, and cannot limit the scope of protection of this disclosure.

## Claims

1. An artificial bone repair material, being a composite material with a porous structure, and raw materials thereof comprising collagen, and porous magnesium-containing tricalcium phosphate powder formed by a precipitate after reaction of calcium salt, magnesium salt and phosphate solution through spray drying, and a particle size of the porous magnesium-containing tricalcium phosphate powder being 1 - 20 µm; wherein, a mass percentage of the porous magnesium-containing tricalcium phosphate to the artificial bone repair material is 50-95%.

2. The artificial bone repair material according to claim 1, is **characterized in that**, a mass ratio of collagen protein to the porous magnesium-containing tricalcium phosphate powder in the raw material is 0.05 to 1.

3. The artificial bone repair material according to claim 1 or 2, is **characterized in that**, the mass percentage of the porous magnesium-containing tricalcium phosphate in the artificial bone repair material is 85-95%, and a porosity of the artificial bone repair material is 70-95%.

4. The artificial bone repair material according to any one of the preceding claims, is **characterized in that**, the artificial bone repair material is formed by crosslinking of the collagen and the porous magnesium-containing tricalcium phosphate powder.

5. The artificial bone repair material according to any one of the preceding claims, is **characterized in that**, a mass percentage of magnesium element in the porous magnesium-containing tricalcium phosphate powder is less than or equal to 3%.

6. The artificial bone repair material according to any one of the preceding claims, is **characterized in that**, the porous magnesium-containing tricalcium phosphate powder is prepared by the following steps:
preparing a mixed solution of calcium salt and magnesium salt as a first solution; preparing a phosphate solution and adjust pH to 9.5-10.5 with alkaline solution as a second solution;
heating and mixing the first solution and the second solution, and reacting under stirring and at 60-95 °C.
centrifuging a suspension obtained after reacting to collect and clean a sediment, and drying cleaned sediment by spray to obtain the porous magnesium-containing tricalcium phosphate powder.

7. A preparation method for an artificial bone repair material, comprising:
dissolving collagen in an acid solution, adding porous magnesium-containing tricalcium phosphate powder, stirring evenly to form a mixed slurry, drying to obtain the artificial bone repair material;
wherein, the porous magnesium-containing tricalcium phosphate powder is obtained by spray-drying a precipitate formed through a reaction of calcium salt, magnesium salt, and phosphate solution, and has a particle size of 1 - 20 µm.

8. The preparation method for an artificial bone repair material according to claim 7, is **characterized in that**, the preparation method comprises the following steps:
S101 - dissolving the collagen in an acid solution, adding the porous magnesium containing tricalcium phosphate powder, stirring evenly, and preparing a mixed slurry;
S102 - adding a crosslinking agent to the mixed slurry, injecting into a molding mold, and freeze drying to obtain a molded composite material;
S103 removing residual crosslinking agent from the composite material using an eluent solution, washing multiple times with pure water, and drying to obtain the artificial bone repair material.

9. The preparation method for an artificial bone repair material according to claim 7 or 8, is **characterized in that**, a mass ratio of collagen and porous magnesium-containing tricalcium phosphate powder is 0.05 - 1; a mass percentage of magnesium element in the porous magnesium-containing tricalcium phosphate powder is less than or equal to 3%.

10. The preparation method for an artificial bone repair material according to any one of claims 7 to 9, is **characterized in that**, the acid solution comprises hydrochloric acid solution or acetic acid solution, with a concentration of 0.1 - 2 wt%, a concentration of collagen in the acid solution is 0.5-5 wt%, the collagen comprises one or a combination of more selected from type I collagen, type II collagen, and recombinant collagen, with a weight average molecular weight of 100000-350000 Da.

11. The preparation method for an artificial bone repair material according to claim 8, is **characterized in that**, the step 102 comprises:
adding 1-100 ppm crosslinking agent to the mixed slurry and injecting into the molding mold, shaking the mold to make slurry liquid level flat, then freezing the mold in an environment of -4 - -80 °C, and then demolding to obtain a molded composite material through freeze-drying method;
wherein, the crosslinking agent includes one or a combination of glutaraldehyde and genipin, and freeze-drying temperature is -20 - -80 °C.

12. The preparation method for an artificial bone repair material according to claim 8 or claim 11, is **characterized in that**, the step 103 comprises:
soaking the composite material in an eluent solution for 0.5-24 hours, placing the eluted composite material in pure water at a speed of 200-600 rpm for stirring and cleaning for 6-24 hours, and then vacuum drying the composite material with a water content greater than 90 wt% to obtain collagen/magnesium containing tricalcium phosphate artificial bone repair material;
wherein, the eluent comprises one or a combination of more selected from polyvinyl alcohol and sodium borohydride, with a concentration of 0.05 - 3 wt%, a vacuum degree for vacuum drying is 1-50 Pa, and a cold trap temperature is -40 - -80 °C.

13. The preparation method for an artificial bone repair material according to any one of claims 7 to 12, is **characterized in that**, the preparation method further comprises a step of preparing the porous magnesium-containing tricalcium phosphate powder, comprising:
preparing a mixed solution of calcium salt and magnesium salt as a first solution, preparing a phosphate solution and adjust pH to 9.5-10.5 with alkaline solution as a second solution;
heating and mixing the first solution and the second solution, and reacting under stirring and at 60-95 °C;
centrifuging a suspension obtained after reacting to collect and clean a sediment, and drying cleaned sediment by spray to obtain the porous magnesium-containing tricalcium phosphate powder.

14. The preparation method for an artificial bone repair material according to claim 13, is **characterized in that**, a molar ratio of calcium ions to magnesium ions in the first solution is 6-10; after mixing the first solution and the second solution, a molar ratio of calcium ions to phosphate ions is 1.1-1.5; the calcium salt comprises one or a combination of more selected from anhydrous calcium chloride, dihydrate calcium chloride, and tetrahydrate calcium nitrate, the magnesium salt comprises magnesium chloride hexahydrate and/or magnesium nitrate hexahydrate, the phosphate salt comprises one or a combination of more selected from disodium hydrogen phosphate, diammonium hydrogen phosphate, and sodium hydrogen phosphate, and the alkaline solution is ammonia water or sodium hydroxide.

15. The preparation method for an artificial bone repair material according to claim 13 or 14, is **characterized in that**, a spray dryer is used to spray dry the washed sediment, an inlet air temperature of the spray dryer is 240 - 300 °C, an outlet air temperature is 120 - 150 °C, and a feed rate is 30 - 200 mL/min.

16. A method for preparing a porous magnesium containing tricalcium phosphate powder for an artificial bone repair material, comprising the following steps:
(i) preparing a mixed solution of calcium salt and magnesium salt as a first solution, preparing a phosphate solution and adjust pH to 9.5-10.5 with alkaline solution as a second solution;
(ii) heating and mixing the first solution and the second solution, and reacting under stirring and at 60-95 °C;
(iii) centrifuging a suspension obtained after reacting to collect and clean a sediment, and spray drying cleaned sediment to obtain the porous magnesium-containing tricalcium phosphate powder.

17. The preparation method according to claim 16, is **characterized in that**, a molar ratio of calcium ions to magnesium ions in the first solution is 6-10.

18. The preparation method of bone repair raw materials according to claim 15, is **characterized in that**, a molar concentration of calcium ions in the first solution is 0.01 - 0.9 mol/L, and a molar concentration of magnesium ions is 0.001 - 0.15 mol/L.

19. The preparation method of bone repair raw materials according to any one of claims 16 to 18, is **characterized in that**, a molar ratio of calcium ions to phosphate ions after mixing the first solution and the second solution is 1.1 to 1.5.

20. The preparation method of bone repair raw materials according to claim 19, is **characterized in that**, a molar concentration of phosphate in the second solution is 0.008-0.8 mol/L.

21. The preparation method of bone repair materials according to any one of claims 16 to 20, is **characterized in that**, the calcium salt comprises one or a combination of more selected from anhydrous calcium chloride, dihydrate calcium chloride, and tetrahydrate calcium nitrate, the magnesium salt comprises hexahydrate magnesium chloride and/or hexahydrate magnesium nitrate, the phosphate salt comprises one or a combination of more selected from disodium hydrogen phosphate, diammonium hydrogen phosphate, and sodium hydrogen phosphate, and the alkaline solution is ammonia water or sodium hydroxide.

22. The preparation method of bone repair material according to any one of claims 16 to 21, is **characterized in that**, in step (i), the pH of the second solution is adjusted to 9.5-10.5 using ammonia water.

23. The preparation method of bone repair material according to any one of claims 16 to 22, is **characterized in that**, in step (ii), the first solution and the second solution are heated to 60-95 °C and mixed.

24. The preparation method of bone repair material according to any one of claims 16 to 23, is **characterized in that**, in step (ii), the stirring rate is 100-500 rpm.

25. The preparation method of bone repair raw material according to any one of claims 16-24, is **characterized in that**, in step (iii), the washed sediment is spray dried with a spray dryer, an inlet air temperature of the spray dryer is 240 - 300 °C, an outlet air temperature is 120 - 150 °C, and the feed rate is 30 - 200 mL/min.

26. The preparation method of bone repair raw material according to claims 16 to 25, is **characterized in that**, in step (iii), the precipitate is added into water for centrifugal cleaning for many times, the washed precipitate is added into water of 5 to 20 times its mass, stirred evenly to form slurry, and then dried by spray.
